# EUROPEAN PATENT SPECIFICATION

(11) **EP 2 702 174 B1**
(45) Date of publication and mention of the grant of the patent: **13.04.2016**
(21) Application number: 12725607.1
(22) Date of filing: 27.04.2012
(51) Int. Cl.: C12Q 1/68

(54) **SYSTEM FOR IDENTIFICATION OF MICRO-ORGANISMS AND DETECTION OF INFECTIOUS DISORDERS**
SYSTEM ZUR IDENTIFIZIERUNG VON MIKRO-ORGANISMEN UND NACHWEIS VON INFEKTIONSERKRANKUNGEN
SYSTÈME D'IDENTIFICATION DE MICRO-ORGANISMES ET DÉTECTION DE TROUBLES INFECTIEUX

(30) Priority: 28.04.2011 DK 201170205
(43) Date of publication of application: 05.03.2014
(73) Proprietor: Zymonostics ApS, 8200 Aarhus N (DK)
(72) Inventor: ANDERSEN, Felicie, F., DK-8000 Aarhus C (DK); KOCH, Jørn, Erland, DK-8660 Ry (DK); STOUGAARD, Magnus, DK-8270 Højbjerg (DK); KNUDSEN, Birgitta, R., DK-8260 Viby J (DK)
(74) Representative: Potter Clarkson LLP
(86) International application number: PCT/DK2012/000056
(87) International publication number: WO 2012/149936

(56) References cited:
- WO-A2-2009/090311
- US-B1- 6 174 678
- ANNAMALAI THIRUNAVUKKARASU ET AL: "Analysis of DNA relaxation and cleavage activities of recombinant Mycobacterium tuberculosis DNA topoisomerase I from a new expression and purification protocol", BMC BIOCHEMISTRY, BIOMED CENTRAL, LONDON, GB, vol. 10, no. 1, 11 June 2009 (2009-06-11), page 18, XP021055571, ISSN: 1471-2091, DOI: 10.1186/1471-2091-10-18
- SISSEL JUUL ET AL: "Detection of Single Enzymatic Events in Rare or Single Cells Using Microfluidics", ACS NANO, AMERICAN CHEMICAL SOCIETY, UNITED STATES, vol. 5, no. 10, 25 October 2011 (2011-10-25), pages 8305-8310, XP002693007, ISSN: 1936-0851, DOI: 10.1021/NN203012Q [retrieved on 2011-09-28]
- SISSEL JUUL ET AL: "Microfluidics-mediated isothermal detection of enzyme activity at the single molecule level", ENGINEERING IN MEDICINE AND BIOLOGY SOCIETY,EMBC, 2011 ANNUAL INTERNATIONAL CONFERENCE OF THE IEEE, IEEE, 30 August 2011 (2011-08-30), pages 3258-3261, XP032025888, DOI: 10.1109/IEMBS.2011.6090885 ISBN: 978-1-4244-4121-1
- CINZIA TESAURO ET AL: "Specific detection of topoisomerase i from the malaria causing P. falciparum parasite using isothermal Rolling Circle Amplification", 2012 ANNUAL INTERNATIONAL CONFERENCE OF THE IEEE ENGINEERING IN MEDICINE AND BIOLOGY SOCIETY, 1 August 2012 (2012-08-01), pages 2416-2419, XP055062200, DOI: 10.1109/EMBC.2012.6346451 ISBN: 978-1-42-444120-4

## Description

### Field of invention

The present invention is defined in the accompanying claims and relates to methods of identifying microorganisms, and methods of diagnosing infectious disorders caused by such microorganisms. Also disclosed herein are methods of treatment and compounds for use in the treatments of such infectious disorders.

### Background of invention

According to WHO, the prevalence of malaria is between 300-500 mill, cases worldwide, while approximately 30% of the human population is expected to be infected with tuberculosis. While malaria primarily affects the poorest regions of the world, tuberculosis (TB) is more widespread in both developing and developed countries. Tuberculosis is a global disease, which pose such a big problem that the World Health Organization in 1993 ruled disaster alarm. The majority of tuberculosis sufferers are found in the third world countries, in particular Africa and Southeast Asia, but there are also cases of tuberculosis in western countries, both among natives and immigrants. The global mobility of tuberculosis is increased due to global traffic and tourism, and the problems of the global prevalence of tuberculosis is underscored by the high prevalence of multidrug-resistant tuberculosis that can not be treated with traditional medicine, in particular the Baltic countries.

On a world wide scale, about two to three million people die every year, in particular children and young people. WHO estimated in 2000 that approx. 33% of the world population (about 2 billion people) is infected with tuberculosis; of these, approx. 5-10% develop the disease at some point in their lifetime.

Tuberculosis is an infectious disease caused by inhalation of tuberculosis bacteria (Mycobacteria tuberculosis). These bacteria attack primarily the lungs, and cause a slight infection during the first six weeks without any serious symptoms. From the lungs, the bacteria can spread through the bloodstream to other organs, although still without necessarily doing any damage at first. In many cases, the infection is fought, if the infected person has a good immune system, however, months or years later, the disease may break out in both lungs and other organs if the immune system is weakened for various reasons. Today, outbreak of tuberculosis often occurs in connection with immune system weakening associated with HIV infection, in particular on the African continent. If tuberculosis is spread further in western countries, tuberculosis outbreak are likely to occur also among cancer patients and other patients, where the immune system is challenged.

A person with active tuberculosis infects on average 10 to 15 other people. Infection occurs through the air with tuberculosis bacteria in saliva droplets from cough or sputum from the patient being inhaled by others. Symptoms of tuberculosis such as heavy coughing and spitting does at least in the initial phases of the disease appear very alarming. The danger of infection is especially high in highly populated areas. The increasing global urbanization combined with increased migration is therefore an important factor in the rising number of tuberculosis cases worldwide.

Among the most important factors in fighting the spread of tuberculosis are effective and rapid methods of diagnosis so that persons with active and infectious tuberculosis can be isolated and subject to treatment. Already after fourteen days of antibiotic treatment, the risk of further transmission of the disease is prevented. To halt the spread of antibiotic resistant tuberculosis bacteria and to curb the spread of infection, WHO recommends a treatment strategy to reduced the DOTS (Directly Observed Treatment Short Course) which provides control and monitoring of patients and as such requires a safe, effective and rapid diagnosis of the disease. One of the problems when it comes to slow the spread of tuberculosis is that it has not yet succeeded in developing diagnosis methods that meet the necessary criteria, such as efficient at-bed-side diagnostic tools. Current diagnosis of TB relies on advanced instrumentation and facilities. Furthermore, diagnostics involve a several day long procedure. The method of the present invention, by contrast, is based on simple technology and can be performed and read-out at the bed-side within a few hours, provided that suitable platform development is achieved. When considering that each untreated TB patient on average transfers the infection to 10-15 other persons, early diagnosis allowing early treatment, which immediately prevents transfer of the disease, is of utmost importance.

### Summary of Invention

The present invention is defined in the accompanying claims. In particular, the present invention is as set out below:
1. A method of identifying a microorganism in a sample, said method comprising:
   i. providing the sample,
   ii. providing a double stranded nucleic acid target specifically for type I topoisomerase of said microorganism, and not for human type I topoisomerase,
   iii. mixing the sample of step i. with the nucleic acid target of step ii.
   iv. detecting nucleic acid target that has been modified by type I topoisomerase of said microorganism,
   wherein the presence of nucleic acid target that has been modified by type I topoisomerase of said microorganism is indicative of said microorganism,
   and wherein said microorganism belongs to the *Plasmodium* genus or the *Mycobacterium* genus.
2. The method according to paragraph 1, wherein said microorganism is a pathogenic microorganism.
3. The method according to any of the preceding paragraphs, wherein said microorganism is *Plasmodium falciparum,* or *Mycobacterium tuberculosis.*
4. The method according to any of the preceding paragraphs, wherein said sample is blood plasma, blood serum, sputum, urine, cell smear or faeces.
5. The method according to any of the preceding paragraphs, wherein said nucleic acid target is a double stranded DNA substrate.
6. The method according to any of the preceding paragraphs, wherein said modified nucleic acid target is detected by southern blotting, polymerase chain reaction, RT-PCR, qPCR, RFLD, primer extension, DNA array technology, rolling circle amplification.
7. The method according to any of the preceding paragraphs, wherein said modified nucleic acid target is detected by rolling circle amplification.
8. A method of determining an infectious disorder in a subject, said method comprising identifying a microorganism in a sample from said subject by a method as defined in any one of paragraphs 1 to 7, wherein the presence of said microorganism in said sample is indicative of said infectious disorder, and wherein said microorganism belongs to the *Plasmodium* genus or the *Mycobacterium* genus.
9. Use of a nucleic acid targeted for a type I topoisomerase of a pathogenic microorganism for diagnosing an infectious disorder, wherein said microorganism belongs to the *Plasmodium* genus or the *Mycobacterium* genus.
10. The method of paragraph 8 or the use according to paragraph 9, wherein said infectious disorder is malaria or tuberculosis.
11. A method for evaluating the effect of an agent on a microorganism in a sample, said method comprising
   i. providing a sample
   ii. providing a double-stranded nucleic acid target specifically for type I topoisomerase of said microorganism, and not for human type I topoisomerase,
   iii. providing an agent,
   iv. combining the sample of step i. and the nucleic acid target of step ii. with or without the agent of step iii.
   v. detecting nucleic acid target that has been modified by type I topoisomerase of said microorganism with or without the agent,
wherein an agent capable of reducing the amount of modified nucleic acid target has an inhibitory effect on said microorganism,
and wherein said microorganism belongs to the *Plasmodium* genus or the *Mycobacterium* genus.

Also described herein but not claimed is an isolated nucleic acid substrate targeted by a type I topoisomerase for use as a diagnosticum for diagnosing an infectious disorder, wherein said nucleic acid substrate is targeted by a type I topoisomerase of a pathogenic microorganism associated with said infectious disorder.

Also described herein but not claimed is a kit comprising a nucleic acid substrate targeted by a type I topoisomerase of a microorganism.

Also described herein but not claimed is a method of treating an infectious disorder, said method comprising administering an agent identified by the screening method above to a subject in need thereof; and an agent identified by the screening method above for use in the treatment of an infectious disorder; and a pharmaceutical composition comprising an agent identified by a method as defined herein, for treating, ameliorating or preventing an infectious disorder.

### Detailed description of the invention

The present invention is defined in the accompanying claims and provides a nucleic acid based biosensor setup with potential use for at-point-of-care diagnosis of infectious disorders, such as malaria or tuberculosis as well as for the fast screening of drugs against the disease-causing *Plasmodium* or *Mycobacterial* pathogens. In the developed setup, specific detection of pathogenic microorganisms, such as malaria parasites, in biological samples, such as crude blood samples, is facilitated by specific enzymatic activities of the pathogenic microorganism, happening within nanometer dimensions, to micrometer-sized products readily detectable at the single molecule level in a fluorescence microscope. A specific example of such enzymatic activity is the conversion of single *P*. *falciparum* topoisomerase I (pfTopl) mediated cleavage-ligation events. This biosensor system requires no special equipment and the readout is adaptable to simple colorimetric detection systems. In addition, the sensitivity of the present biosensor setup is clearly superior to standard cold immuno-based diagnostics. Therefore, the presented biosensor is an easy-to-use diagnostic tool, suitable even for the malaria epidemic areas in developing countries.

The present invention relates to methods of identifying a microorganism, and methods of diagnosing infectious disorders caused by such microorganism as defined by the claims. Also described herein but not claimed are methods of treatment and compounds for use in the treatments of such infectious disorders. Generally, the microorganism is identified on the basis of its expression of a DNA modifying enzyme, which is specific for that particular microorganism, in particular DNA modifying enzymes which display a site-specific DNA modifying activity. In this way, specific nucleic acid substrates are employed, which comprise a sequence specifically targeted by the enzymes in question, where the processing of that substrate is indicative of the presence of that particular microorganism.

Described herein is a generic platform for detecting any organism that expresses its own variant of a DNA modifying enzyme. The concept of the invention extends to any enzyme system, such as nucleases, phosphatises, phosphorylases, topoisomerases and others, including DNA modifying enzymes systems, where a cascade of enzymes works to modify a nucleic acid target.

The method of the present invention is highly sensitive and simple, and requires only a short reaction time before an answer is obtained with respect to the presence of a microorganism.

### Definitions

To facilitate the understanding of the invention, some definitions of important terms are provided herein below.

As used herein, *"nucleic acid*" or "*polynucleotide*" refers to polynucleotides, such as deoxyribonucleic acid (DNA) or ribonucleic acid (RNA), oligonucleotides, fragments generated by the polymerase chain reaction (PCR), and fragments generated by any of ligation, scission, endonuclease action, and exonuclease action. Polynucleotides can be composed of monomers that are naturally-occurring nucleotides (such as DNA and RNA), or analogs of naturally-occurring nucleotides (e.g., (alpha-enantiomeric forms of naturally-occurring nucleotides), or a combination of both. Modified nucleotides can have alterations in sugar moieties and/or in pyrimidine or purine base moieties. Sugar modifications include, for example, replacement of one or more hydroxyl groups with halogens, alkyl groups, amines, and azido groups, or sugars can be functionalized as ethers or esters. Moreover, the entire sugar moiety can be replaced with sterically and electronically similar structures, such as aza-sugars and carbocyclic sugar analogs. Examples of modifications in a base moiety include alkylated purines and pyrimidines, acylated purines or pyrimidines, or other well-known heterocyclic substitutes. Nucleic acid monomers can be linked by phosphodiester bonds or analogs of such linkages. Analogs of phosphodiester linkages include phosphorothioate, phosphorodithioate, phosphoroselenoate, phosphorodiselenoate, phosphoroanilothioate, phosphoranilidate, phosphoramidate, and the like. The term "polynucleotide" also includes so-called "peptide nucleic acids," which comprise naturally-occurring or modified nucleic acid bases attached to a polyamide backbone. Nucleic acids can be either single stranded or double stranded.

The term "complement" in terms of a nucleic acid sequence refers to a polynucleotide having a complementary nucleotide sequence and reverse orientation as compared to a reference nucleotide sequence. For example, the sequence 5' ATGCACGGG 3' is complementary to 5' CCCGTGCAT 3'.

"Complementary DNA (cDNA)" is a single-stranded DNA molecule that is formed from an mRNA template by the enzyme reverse transcriptase. Typically, a primer complementary to portions of mRNA is employed for the initiation of reverse transcription. Those skilled in the art also use the term "cDNA" to refer to a double-stranded DNA molecule consisting of such a single-stranded DNA molecule and its complementary DNA strand. The term "cDNA" also refers to a clone of a cDNA molecule synthesized from an RNA template.

The term *'nucleotides'* as used herein refers to both natural nucleotides and *non-*natural nucleotides, which are capable of being incorporated into an oligonucleotide, such as a splice-switching oligonucleotide. Nucleotides may differ from natural nucleotides by having a different phosphate moiety, sugar moiety and/or base moiety. Nucleotides may accordingly be bound to their respective neighbour(s) in a template or a complementing template by a natural bond in the form of a phosphodiester bond, or in the form of a non-natural bond, such as e.g. a peptide bond as in the case of PNA (peptide nucleic acids).

### Sequence identity

The term "sequence identity" indicates a quantitative measure of the degree of homology between two amino acid sequences or between two nucleic acid sequences of equal length. If the two sequences to be compared are not of equal length, they must be aligned to give the best possible fit, allowing the insertion of gaps or, alternatively, truncation at the ends of the polypeptide sequences or nucleotide sequences. The sequence identity can be calculated, wherein Ndif is the total number of non-identical residues in the two sequences when aligned and wherein Nref is the number of residues in one of the sequences, preferably sequence identity is calculated over the full length reference as provided herein. Hence, the DNA sequence AGTCAGTC will have a sequence identity of 75% with the sequence AATCAATC (Ndif=2 and Nref=8). A gap is counted as non-identity of the specific residue(s), i.e. the DNA sequence AGTGTC will have a sequence identity of 75% with the DNA sequence AGTCAGTC (Ndif=2 and Nref=8).

With respect to all embodiments of the invention relating to nucleotide sequences, the percentage of sequence identity between one or more sequences may also be based on alignments using the clustalW software (http:/www.ebi.ac.uk/clustalW/index.html) with default settings. For nucleotide sequence alignments these settings are: Alignment=3Dfull, Gap Open 10.00, Gap Ext. 0.20, Gap separation Dist. 4, DNA weight matrix: identity (IUB).

The following terms are used to describe the sequence relationships between two or more polynucleotides: "predetermined sequence", "comparison window", "sequence identity", "percentage of sequence identity", and "substantial identity".

A "predetermined sequence" is a defined sequence used as a basis for a sequence comparison; a predetermined sequence may be a subset of a larger sequence, for example, as a segment of a full-length DNA or gene sequence given in a sequence listing, such as a polynucleotide sequence as disclosed herein or may comprise a complete DNA or gene sequence. Generally, a predetermined sequence is at least 20 nucleotides in length, frequently at least 25 nucleotides in length, and often at least 50 nucleotides in length. Likewise, the predetermined sequence may refer to polypeptides.

The term "diagnosticum" refers in the present context to a compound or composition used in diagnosis of a disease or medical state. In the present text the diagnosticum is a binding member or a detection member of the present invention or active derivative thereof for use in the diagnosis of a disease or condition, as described herein above.

The nucleic acid substrate of the invention may be used as a diagnosticum, as defined in the accompanying claims.

### Nucleic acid modifying enzyme system

By definition enzymes convert substrate molecules to products with changed chemical or physical characteristics without being affected by the process. Hence, at least theoretically one enzyme can create indefinite amounts of product provided with sufficient substrates and, consequently, the most sensitive detection of pathogens imaginable relies on detection of species-specific enzymatic products.

According to the methods and uses of the present invention defined in the claims, a microorganism is identified in a sample by detecting a nucleic acid substrate which is targeted by a nucleic acid modifying enzyme system specific for said microorganism. This detection method also forms the basis of the diagnostic methods, compositions and uses of the present invention defined in the claims.

Also described herein but not claimed are products, methods and uses that extend to any enzyme system, such as nucleases, phosphatises, phosphorylases and others, and nucleic acids modifying enzymes system where a cascade of enzymes works to modify a nucleic acid target.

### Type I topoisomerase

As defined in the claims, the present invention relates to nucleic acid-based detection assays based on type 1 topoisomerase for the identification of a microorganism via the detection of specific single enzymatic products mediated by topoisomerase I. In general, type I topoisomerases act by introducing single strand cuts in DNA followed by subsequent ligation of the generated nick in a reaction that involves the formation of a covalent enzyme-DNA cleavage intermediate.

As defined in the claims, the invention involves identification of a microorganism in a sample by detecting a nucleic acid substrate which is targeted by a type I topoisomerase of said microorganism. Type I topoisomerase targets double stranded nucleic acid molecules by binding a region of said nucleic acid molecule and cleaving a single strand of the duplex.

The cleavage reaction of type I topoisomerase can be conducted on a specific nucleic acid substrate, which upon cleavage is converted from a self-folding so-called dumbbell substrate to a closed nucleic acid circle. That circle may then subsequently be subjected to Rolling Circle Amplification (RCA) leading to a product (RCP) consisting of ~10³tandem repeats of a sequence complementary to the DNA circles. Each RCP can be visualised at the single-molecule level in a fluorescence microscope by annealing to fluorescent-labelled probes giving rise to one fluorescent spot for each RCP. Since rolling circle amplification involves no thermal cycling, each RCP represents one closed DNA circle, which in turn represents a single cleavage-ligation event. False positives are avoided by depleting the reaction buffers for divalent cations, which is a prerequisite for the activity of most DNA modifying enzymes, including ligases, but not for type I topoisomerases such as pf-topoisomerase I and tuberculosis topoisomerase I.

### Biosensor for detection of tuberculosis

Topoisomerase I from the tuberculosis-causing pathogen M. tuberculosis (MtTopl) belongs to the typelA family of topoisomerases, which normally require Mg2+ for activity. However, MtTopl only requires this cofactor during the ligation step of catalysis and not during cleavage. Therefore, MtTopl cleavage can be detected even in crude biological samples, which are depleted for Mg2+. Moreover, MtTopl cleaves single stranded DNA in a sequence specific manner, which allows the specific cleavage activity of MtTopl to be distinguished from other nucleases. Hence, a single stranded nucleic acid substrate is provided to the sample to be tested for *Mycobacterium tuberculosis,* and that nucleic acid substrate is then upon cleavage by MtTopl converted to a well-defined product with a specific sequence. This product can then be detected by any suitable method.

For example, the cleavage product is hybridized to a primer anchored to a glass surface and circularized by a DNA ligase (in the presence of Mg2+) after cell remains have been washed away. The generated circle can now serve a template for RCA and the products visualized by hybridization to specific fluorescent probes (cf. figure 10).

### Nucleic acid substrate

The nucleic acid substrate may be labelled, and/or hybridized to one or more nucleic acid probes, and detected via the respective label. The nucleic acid substrates may be coupled to a support. Such supports are well known to those of ordinary skill in the art and include, but are not limited to glass, plastic, metal, or latex. In particular aspects of the invention, the support can be planar or in the form of a bead or other geometric shapes or configurations known in the art.

### Identification of a microorganism

As defined in the claims, the present invention relates to a method of identifying a microorganism in a sample.

The microorganism is preferably a pathogenic microorganism, which is involved in malaria or tuberculosis, for example, (*Plasmodium falciparum* or *Mycobacterium tuberculosis.)*

In a more specific application, the microorganism is identified in a sample from a human subject. The nucleic acid substrate is targeted specifically by the microorganism and not by human topoisomerase I.

### Sample

A "sample" according to the present invention is any suitable biological sample. The biological sample is in a preferred embodiment, isolated from a subject, such as a human being. The choice of sample depends on the specific microorganism or infectious disorder to be determined as well as the detection method, and will be appreciated by those of skill in the art. In one embodiment the sample is a blood sample, a tissue sample, a secretion sample, semen, ovum, hairs, nails, tears, urine or faeces. A convenient sample type is a blood sample. The blood sample includes any fraction of blood, such as blood plasma or blood serum, sputum, urine, cell smear.

However, the sample of the invention may also be a tissue sample, such as a sample of a tissue selected from the group consisting of skin, epidermis, dermis, hypodermis, breast, fat, thymus, gut, small intestine, large intestine, stomach, muscle, pancreas, heart muscle, skeletal muscle, smooth muscle, liver, lung, brain, cornea and tumours, ovarian tissue, uterine tissue, colon tissue, prostate tissue, lung tissue, renal tissue, thymus tissue, testis tissue, hematopoietic tissue, bone marrow, urogenital tissue, expiration air, stem cells, including cancer stem cells

### Microorganism

The present invention relates to a method for identifying a microorganism as defined in the claims. Also described herein are methods and compounds for treating an infectious disorder, which is caused by a microorganism. The microorganism of the invention is thus, mostly, a pathogenic microorganism.

Microorganism includes bacteria and viruses.

The microorganism identified by the method of the present invention is for example involved in and/or is the causative agent in malaria or tuberculosis. Also described herein but not claimed are methods in which the microorganism is for example involved in toxoplasmosis or Lyme disease/borreliosis (Borrelia).

In methods described herein but not claimed, the microorganism is enterobacteria, enterococci, corynebacteria, Salmonella spp, Brachyspira hyodysenteriae, Lawsonia intracellularis, campylobacter spp., clostridia, coronavirus, rotavirus, torovirus, calicivirus, astrovirus, canine parvovirus, coccidia and cryptosporidia, E. coil, Salmonella spp, Yersinia spp., including Yersiriia enterocolitica, Coxiella burnetti" rotavirus, coronavirus, calicivirus, bovine virus diarrhoea virus, bovine herpes virus, rinderpest virus, coccidia, and cryptosporidia, Salmonella spp, Lawsonia intracellularis, Campylobacter spp, Enteropathogenic E.coli, Brachyspira spp including Brachyspira hyodysenteria, Clostridium spp, rotavirus, sappovirus, norovirus, and coronavirus, Salmonella spp, Camphylobacter spp.,Norovirus, rotavirus, Vibrio spp. including Vibrio cholera, Shigella spp., Helicobacter spp., coccidia or cryptosporidia.

The microorganism belongs to the Plasmodium genus. The microorganism is for example selected from the following species: Plasmodium clelandi, Plasmodium draconis, Plasmodium lionatum, Plasmodium saurocordatum, Plasmodium vastator, Plasmodium juxtanucleare, Plasmodium basilisci, Plasmodium Plasmodium cyclopsi, Plasmodium foleyi, Plasmodium girardi, Plasmodium incertae, Plasmodium inopinatum, Plasmodium landauae, Plasmodium lemuris, Plasmodium melanipherum, Plasmodium narayani, Plasmodium odocoilei, Plasmodium percygarnhami, Plasmodium pulmophilium, Plasmodium sandoshami, Plasmodium traguli, Plasmodium tyrio, Plasmodium uilenbergi, Plasmodium vinckei, Plasmodium watteni and Plasmodium yoelli.

Most preferred, the microorganism is Plasmodium falciparum, which is a causative agent of Malaria.

In another preferred embodiment, the microorganism belongs to the Mycobacterium genus. The microorganism is for example selected from the Mycobacterium tuberculosis complex (MTBC), the members of which are causative agents of human and animal tuberculosis. Species in this complex include: M. tuberculosis, M. bovis, M. bovis BCG, M. africanum, M. canetti, M. caprae, M. microti, Mycobacterium avium sp. paratuberculosis and M. pinnipedii. Most preferrably, the microorganism is Mycobacterium tuberculosis, which is the major cause of human tuberculosis.

### Detection

According to the methods and uses of the present invention, a microorganism is identified in a sample by detecting a nucleic acid substrate which is targeted by a type I topoisomerase of said microorganism as defined in the claims. As described above, type I topoisomerase targets double stranded nucleic acid molecules by binding a region of said nucleic acid molecule and cleaving a single strand of the duplex. A nucleic acid substrate, which has been targeted by type I topoisomerase may thus be detected by identifying those nucleic acid substrates in the sample that have been cleaved. The nucleic acid substrate is thus, preferably targeted by a topoisomerase I of the microorgansms only, and not by other topoisomerase I-activities of the sample, such as native topoisomerases of the subject tested for microorganisms or infectious disorders such as malaria and/or tuberculosis.

Detection of cleaved and uncleaved (targeted and untargeted) nucleic acid substrates may be performed by any suitable method available. Detection is for example obtained by southern blotting, polymerase chain reaction, RT-PCR, qPCR, RFLD, primer extension, DNA array technology, rolling circle amplification. In a preferred embodiment, the nucleic acid substrate is detected by rolling circle amplification, for example by a method as described in WO 2008/148392.

In certain aspects, a detection assay can be a quantitative amplification assay, such as quantitative PCR (qPCT) or quantitative RT-PCR (qRT-PCR). Other methods include hybridization assays, such as array hybridization assays or solution hybridization assays. The nucleic acid substrate may be labelled, and/or hybridized to one or more nucleic acid probes, and detected via the respective label.

In a convenient setup of the detection methods of the present invention, a simple portable readout devices or even with calorimetric readout visible for the naked eye, adapting the biosensor for at-place-of-care diagnosis suitable even for the special requirements of third world countries currently suffering the major burden of malaria epidemics.

### Applications

The invention defined in the claims may be employed for identifying microorganism and/or an infection in any subject, including humans, non-human animals, such as house-hold stocks, and plants. Other applications of the method include the identification of microorganisms in the contamination of food and drinking water.

### Medical use

The compositions, kits and methods provided herein are also intended for medical use. Specifically, the compositions, methods and medicaments are provided for identifying a microorganism as defined in the claims. The microorganisms as defined in the claims are preferably pathogenic microorganism, i.e. are among the causative agents of an infectious disorder. Therefore, the compositions, kits and methods of the present invention are also provided for the diagnosis of infectious disorders as defined in the claims, in particular for the diagnosis of malaria and/or tuberculosis

In a further aspect as defined in the claims, the present invention relates to a method of identifying novel lead compounds for treatment of infectious disorders. To this end, a method is provided for evaluating the effect of an agent on a pathogenic microorganism. Based on the compounds identified in such method, the present invention also relates to such agents for use in the treatment of an infectious disorder, in particular malaria and/or tuberculosis.

### Diagnosis

So in one main medical aspect, the present invention relates to a method of determining an infectious disorder in a subject, in particular in a human being. As defined in the claims, the method comprises identifying a microorganism in a sample from said subject by a method of the present invention; the presence of microorganism in said sample is then indicative of said infectious disorder, because the microorganism is a causative agent of that particular infectious disorder. The infectious disorder determined according to the present invention is for example without limitation tuberculosis, malaria, toxoplasmosis or Lyme disease/borreliosis (Borrelia).

A large number of microorganisms are known to be associated as causative agents with certain infectious disorders. For example, *Plasmodium falciparum* is known to be a causative agent of malaria, and *Mycobacterium tuberculosis* is known as a major causative agent of human tuberculosis.

The diagnostic applications of the present invention may be practised in any suitable and practical setup or machinery, which utilizes the system's sensitivity, simplicity and short reaction time. For example, the method may be used in advanced equipment for single cell, single molecule detection, for ultra-sensitive detection of infection sources.

However, in a particularly preferred embodiment, the diagnostic methods are performed in the style of stick tests/dipsticks. A testing dipstick is for example made of paper or cardboard and is impregnated with the reagents required to perform the reaction of the invention. The readout of a dipstick test is preferably presented by a changing color. In this way, dipsticks can be used to test for a variety of liquid samples for the presence of a specific microorganism as defined in the claims, and the dipstick can then be employed in easy and efficient diagnosis of infectious disorders, such as any infectious disorder according to the present invention.

### Method for drug discovery

The species-specific enzyme reactions, particular type I topoisomerase activity, which serve to modify nucleic acids/DNA, and thereby are used for identification of a microorganism, are generally essential for these microorganisms since they are part of DNA metabolism. Any compound capable of specifically blocking, inhibiting or down-regulating the activity of these species-specific enzymes may be used as therapeutic against such microorganisms and/or infectious disorders caused by such microorganisms. The methodology of the present invention can be applied directly to the testing of drugs known for their selective action on specific enzymatic processes in the relevant microorganism. Moreover, the methodology can be used for design of new small molecule drugs against nucleic acids modifying enzyme systems of infectious microorganisms. Accordingly, the method defined in the claims may also be employed in drug discovery, because the method provided herein for determining the presence of microorganisms via the present of a specific type I topoisomerase activity, can be used for evaluating the effect of a candidate drug on a microorganism. Any such drug candidates, which display an inhibitory effect on the present of the microorganism and/or on the activity of the toposimerase activity of said microorganism is a suitable drug for treatment of an infectious disorder associated with that microorganism.

Also described herein but not claimed is an agent identified by a of the present invention and/or a pharmaceutical composition comprising such agent for use in the treatment, prevention or amelioration of an infectious disorder.

### Examples

### Development of a Novel Plasmodium falciparum Topoisomerase I Specific Biosensor for Diagnosis of Malaria

This example relates to a DNA based biosensor suitable for at-point-of-care diagnosis of malaria. In this setup, specific detection of malaria parasites in crude blood samples is facilitated by the conversion of single Plasmodium falciparum topoisomerase I (pfTopl) mediated cleavage-ligation events, happening within nanometer dimensions, to micrometer-sized products readily detectable at the single molecule level in a fluorescence microscope.

One challenge of detecting enzymatic products is that only few enzymatic products are readily detectable without the use of sophisticated equipment and even then, most products can be detected only when produced in high numbers. For clinically relevant identification of pathogens based on species specific enzymatic activities it is, therefore, necessary to have detection systems that overcome these challenges.

The enzymes Topoisomerase I (hTopl), Flp and Cre all introduce single strand cuts in DNA followed by subsequent ligation of the generated nick in a reaction that involves the formation of a covalent enzyme-DNA cleavage intermediate. This reaction may be utilized to convert self-folding oligonucleotide substrates to closed DNA circles, which subsequently were subjected to Rolling Circle Amplification (RCA) leading to products (RCP) consisting of ∼10³ tandem repeats of a sequence complementary to the DNA circles. These RCPs can be visualised at the single-molecule level in a fluorescence microscope by annealing to fluorescent-labelled probes giving rise to one fluorescent spot for each RCP. Since the assay involvs no thermal cycling, each RCP represents one closed DNA circle, which in turn represented a single cleavage-ligation event. Hence, this assay allows the detection of Topl, Flp or Ore activity at the single cleavage-ligation event level.

Here, the assay is used for identification of the malaria parasite P. falciparum in crude clinical samples based on the specific detection of single pfTopl cleavage-ligation events. First a synthetic gene encoding pftopl was cloned and the recombinant protein expressed in and purified from Saccharomyces Cerevisiae to allow characterization of the enzyme. The ability of pfTopl to cleave the classical hexadecameric sequence known as a preferred cleavage site for most other nuclear typelB topoisomerases was investigated using a synthetic 75-mer substrate with this sequence. pftopl cleaved this substrate between nucleotides -1 and +1, which is the preferred cleavage site for other nuclear typelB topoisomerases, including hTopl, as well as several addition sites located downstream to this position, which is not cleaved by hTopl. Based on this result it was anticipated that single cleavage-ligation events mediated by pfTopl could be detected in an RCA-based biosensor system using the substrate (Sul) originally developed to detect hTopl activity. This expectation held true. Moreover, since pfTopl exhibits a considerably higher salt tolerance than does hTopl increasing the salt concentration enabled the specific detection of pfTopl on a background of human cell content including hTopl in extracts from cell lines or human blood. However, the specific detection of pfTopl obtained in this manner was at the cost of sensitivity, with salt (400-500 mM) concentrations high enough to prevent hTopl activity decreasing pfTopl activity.

in contrast to its human counterpart, pfTopl is able to cleave close to DNA ends with high efficiency. Hence, a DNA substrate which is circularized upon cleavage-ligation close to a DNA end may enable specific detection of pfTopl on a background of the human cell extract without compromising sensitivity of the assay considerably. To address this possibility, purified pfTopl was incubated with each of the substrates Su2-Su6 and the products analysed using the RCA based detection system. The sequence of the substrates Su4, Su5, and Su6 was modified to match the sequence that was cleaved with high efficiency in substrate XX. pfTopl was able to convert Su2-Su6 to closed circles readily detectable in the RCA-based biosensor setup, while hTopl was not. Of the different substrates and assay conditions tested out the utilization of Su2 appeared the most efficient for specific detection of pfTopl.

The use of Su2 for detecting pfTopl activity in human cell extracts in the RCA-based biosensor setup was also verified. For this purpose nuclear extracts from HEK-293T cells were incubated with Su1 (which is circularized by hTopl and serve as a control of efficient cell lysis) and Su2 before or after addition of spike-in purified pfTopl followed by RCA and visualization of RCPs as outlined in Fig1 B. As a control for successful RCA and probe annealing a circularized control circle was added to each sample before annealing to the primer coated slide. Red spots corresponding to RCPs originating from Su2 were only observed upon addition of spike-in pfTopl to the extract, verifying that Su2 serves as a substrate specific for pfTopl even in crude cell extracts. As expected, comparative levels of green and blue spots corresponding to RCPs from Su1 and control circles, respectively, could be observed in both samples.

To test the use of pfTopl specific RCA-based detection setup for diagnosis of malaria, extracts from either non-infected or in vitro generated P. falciparum infected human, Red Blod Cells (RBC) were subjected to analysis. Consistent with Su2 being circularized only by pfTopl red signals originating from RCPs of this substrate were observed only after incubation with extracts from P. falciparum infected RBC, whereas signals originating from RCPs of circularized Su1 or the control circle could be observed upon incubation with extracts from both uninfected and infected RBC. Note that the hTopl activity observed in extract from infected RBC was considerably lower than in extract from noninfected RBC. Since the same cell extracts were used in both experiments we believe this to be a side effect of cells suffering in different ways from the P. falciparum infection. A similar result was obtained when analysing extracts from a blood sample from a mildly infected malaria patient, further verifying the validity of the detection method to specifically detect the presence of P. falciparum parasites in clinical relevant samples

The presented method allows the detection of down to 2x104 parasites/p1 of RBC. In comparison the detection limit of PCR using standard primers specific for Plasmodium sp. or P. falciparum specific genomic sequences was around 1 parasite/µlof RBC, whereas the detection limit of a commercially available malaria RDT was about XX parasites/µl. Note, that although PCR is by several orders of magnitude more sensitive than the RCA-based biosensor, at least in its current crude setup, this technique do not allow a quantitative estimation of the infection level, which is possible with RCA-based biosensor. Moreover, the PCR analyses required purification and concentration of genomic DNA to perform, whereas the biosensor allowed P. falciparum detection directly in crude cell extracts. Regarding sensitivity the presented RCA-biosensor by far outcompetes current state of the art malaria RDT.

In conclusion, the present example demonstrates the specific, easy and sensitive detection of malaria in clinical relevant samples by visualizing single cleavage-ligation events mediated by pfTopl. This is achieved by a special developed biosensor system in which each catalytic reaction by pfTopl is converted to a micrometer-sized product readily visible at the single-molecule level. Since each pfTopl, potentially can perform thousands of catalytic reactions without losing activity, the sensitivity of the biosensor is would outcompete current immunohistochemical based diagnostic tools and may allow diagnosis based on non-invasive samples such as mucus or saliva, which typically contain only sparse numbers of P. falciparum parasites. This can be achieved by concentrating the RCP signals. Note, that concentrating RCPs on sequencing beads significantly improve sensitivity of the assay. With regard to handling and speed, the present method is superior PCR, and provides a quantitative measurement allowing continuous monitoring of disease development and treatment, which PCR cannot provide.

### Sequences

Mycobacterium topoisomerase I gene:

TTGGCTGACCCGAAAACGAAGGGCCGTGGCAGCGGCGGCAATGGCAGCGGCCGGCG
ACTGGTCATCGTCGAGTCGCCCACCAAGGCGCGCAAGCTGGCCTCCTACCTGGGCTC
TGGCTACATCGTCGAGTCCTCCCGGGGGCACATCCGTGACTTGCCGCGGCCGCGTC
GGATGTACCCGCAAAGTACAAGTCGCAGCCGTGGGCGCGGCTCGGGGTCAACGTCG
ACGCCGACTTCGAACCGCTCTACATCATCAGCCCGGAGAAACGGAGCACCGTCAGCG
AGCTCAGGGGCCTGCTCAAAGACGTGGACGAGCTGTATCTGGCCACGGATGGGGAC
CGTGAGGGCGAAGCTATTGCCTGGCATCTGCTGGAAACCCTCAAACCGCGCATACCG
GTAAAGCGGATGGTCTTCCACGAGATCACCGAACCGGCGATCCGCGCCGCCGCCGA
GCACCCCCGCGACCTAGACATCGACCTGGTCGACGCGCAGGAGACCCGGCGCATCC

TGGACCGGCTGTACGGCTACGAAGTCAGCCCAGTGCTGTGGAAGAAGGTCGCCCCC
AAGTTGTCGGCGGGCCGGGTGCAGTCGGTGGCCACCCGCATCATCGTGGCGCGCGA
ACGCGACCGCATGGCGTTCCGCAGCGCGGCCTACTGGGACATCCTTGCCAAGCTGG
ATGCCAGCGTGTCCGACCCGGACGCCGCGCCGCCCACCTTCAGCGCCCGGCTGACG
GCCGTGGCTGGCCGGCGGGTGGCCACTGGCCGCGATTTCGACTCGCTGGGCACGCT
GCGCAAAGGCGACGAAGTCATTGTGCTCGACGAGGGAGCGCGACCGCGTTGGCCG
CGGGCCIGGATGGCACGCAGCTGACCGTGGCCTCGGCCGAGGAGAAGCCCTACGCC
CGGCGCCCGTACCCGCCGTTCATGACCTCCACGCTGCAGCAAGAGGCCAGCCGCAA
GCTGCGGTTCTCCGCCGAGCGGACGATGAGCATCGCCCAGCGGCTGTACGAAAACG
GCTACATCACCTATATGCGTACCGACTCCACCACGCTGTCGGAGTCGGCGATCAACGC
CGCACGTACCCAGGCGCGCCAGCTCTACGGCGACGAGTACGTCGCGCCGGCGCCGC
GCCAATACACCCGCAAGGTGAAGAACGCCCAGGAAGCGCACGAGGCTATCCGGCCC
GCCGGTGAAACGTTTGCCACCCCGGACGCGGTGCGTCGCGAACTCGACGGTCCCAAC
ATTGATGATTTCCGGCTCTATGAGCTGATTTGGCAACGCACCGTAGCCTCGCAGATGG
CCGATGCGCGGGGCATGACGCTGAGCCTGCGGATCACTGGCATGTCGGGGCACCAG
GAGGTGGTGTTCTCCGCGACCGGACGCACCTTGACGTTCCCGGGCTTCCTCAAGGCC
TACGTGGAGACCGTGGACGAGCTGGTCGGCGGCGAGGCTGACGATGCCGAGCGGCG
ACTGCCCCATCTGACCCCGGGTCAACGGTTGGACATCGTCGAGTTGACCCCAGACGG
CCATGCCACCAACCCGCCGGCCCGCTACACCGAGGCGTCGCTGGTCAAAGCGCTCGA
GGAGCTGGGCATCGGCCGCCCGTCGACCTACTCGTCGATCATCAAGACCATCCAGGA
TCGCGGCTACGTGCACAAGAAGGGCAGTGCACTGGTGCCGTCATGGGTGGCGTTCG
CGGTAACCGGICTGCTCGAGCAGCATTTCGGTCGGCTCGTCGACTACGACTTCACCG
CGGCGATGGAAGACGAGCTCGACGAGATCGCCGCCGGCAACGAGCGCCGCACCAAC
TGGCTCAACAACTTCTACTTTGGIGGCGATCACGGTGTGCCCGATTCGGTAGCCCGAT
CGGGTGGCCTCAAGAAGCTTGTCGGGATCAATCTCGAGGGCATCGACGCACGAGA
AGTAAACTCTATCAAGCTTTTTGACGACACCCACGGACGCCCCATATATGTTCGGGT
GGGCAAGAACGGTCCCTACCTGGAACGTTTGGTGGCCGGCGACACCGGTGAGCCCA
CGCCGCAGCGGGCCAACCTCAGCGACTCGATTACCCCGGACGAGCTGACTCTACAG
GTGGCCGAAGAGCTCTTTGCCACACCGCAACAGGGACGGACTTTGGGCTTGGACCCA
GAAACCGGCCACGAGATCGTGGCCAGGGAAGGCCGGTTTGGGCCGTATGTGACCGA
GATCCTGCCGGAGCCTGCGGCTGATGCGGCCGCGGCCGCTCAGGGAGTCAAGAAA
CGCCAGAAGGCCGCCGGGCCCAAACCGCGCACCGGTTCGTTGCTGCGGAGCATGG
ACCTACAGACGGTCACCCTCGAAGACGCGCTGAGGCTGCTGTCACTGCCGCGCGTG
GTCGGAGTGGACCCCGCCTCGGGTGAGGAGATCACCGCGCAGAACGGGCGCTACG
GACCGTATCTAAAGCGCGGCAACGATTCTCGATCACTGGTCACCGAAGACCAGATAT
TCACCATCACGCTCGACGAAGCCCTGAAGATCTACGCAGAGCCGAAACGTCGTGGC
CGGCAAAGCGCTTCGGCTCCGCCGCTGCGCGAGCTGGGAACAGATCCGGCGTCGG

GCAAGCCAATGGTCATCAAGGACGGCCGATTCGGGCCGTACGTCACCGACGGTGAG
ACCAATGCCAGCCTGCGTAAGGGCGACGACGTGGCTTCCATAACCGACGAGCGCGC
CGCCGAGCTGTTGGCCGATCGCCGAGCCCGGGGTCCGGCAAAACGGCCAGCCAGG
AAAGCTGCCCGGAAGGTGCCGGCGAAGAAGGCAGCCAAGCGCGACTAG

Mycobacterium topoisomerase I protein:

MADPKTKGRGSGGNGSGRRLVIVESPTKARKLASYLGSGYIVESSRGHIRDLPRAAS
DVPAKYKSQPWARLGVNVDADFEPLYIISPEKRSTVSELRGLLKDVDELYLATDGDREGEA
IAWHLLETLKPRIPVKRMVFHEITEPAIRAAAEHPRDLDIDLVDAQETRRILDRLYGYEVSPV
LWKKVAPKLSAGRVQSVATRIIVARERDRMAFRSAAYWDILAKLDASVSDPDAAPPTFSAR
LTAVAGRRVATGRDFDSLGTLRKGDEVIVLDEGSATALAAGLDGTQLTVASAEEKPYARR
PYPPFMTSTLQQEASRKLRFSAERTMSIAQRLYENGYITYMRTDSTTLSESAINAARTQAR
QLYGDEYVAPAPRQYTRKVKNAQEAHEAIRPAGETFATPDAVRRELDGPNIDDFRLYELIW
QRTVASQMADARGMTLSLRITGMSGHQEVVFSATGRTLTFPGFLKAYVETVDELVGGEA
DDAERRLPHLTPGQRLDIVELTPDGHATNPPARYTEASLVKALEELGIGRPSTYSSIIKTIQD
RGYVHKKGSALVPSWVAFAVTGLLEQHFGRLVDYDFTAAMEDELDEIAAGNERRTNWLN
NFYFGGDHGVPDSVARSGGLKKLVGINLEGIDAREVNSIKLFDDTHGRPIYVRVGKNGP
YLERLVAGDTGEPTPQRANLSDSITPDELTLQVAEELFATPQQGRTLGLDPETGHEIVAR
EGRFGPYVTEILPEPAADAAAAQGVKKRQKAAGPKPRTGSLLRSMDLQTVTLEDALRLLS
LPRVVGVDPASGEEITAQNGRYGPYLKRGNDSRSLVTEDQIFTITLDEALKIYAEPKRRGRQ
SASAPPLRELGTDPASGKPMVIKDGRFGPYVTDGETNASLRKGDDVASITDERAAELLADR
RARGPAKRPARKAARKVPA K K A A K R D

## Claims

1. A method of identifying a microorganism in a sample, said method comprising:
i. providing the sample,
ii. providing a double stranded nucleic acid target specific for type I topoisomerase of said microorganism, and not for human type I topoisomerase,
iii. mixing the sample of step i. with the nucleic acid target of step ii.
iv. detecting nucleic acid target that has been modified by type I topoisomerase of said microorganism,
wherein the presence of nucleic acid target that has been modified by type I topoisomerase of said microorganism is indicative of said microorganism,
and wherein said microorganism belongs to the *Plasmodium* genus or the *Mycobacterium* genus.

2. The method according to claim 1, wherein said microorganism is a pathogenic microorganism.

3. The method according to any of the preceding claims, wherein said microorganism is *Plasmodium falciparum,* or *Mycobacterium tuberculosis.*

4. The method according to any of the preceding claims, wherein said sample is blood plasma, blood serum, sputum, urine, cell smear or faeces.

5. The method according to any of the preceding claims, wherein said nucleic acid target is a double stranded DNA substrate.

6. The method according to any of the preceding claims, wherein said modified nucleic acid target is detected by southern blotting, polymerase chain reaction, RT-PCR, qPCR, RFLD, primer extension, DNA array technology, rolling circle amplification.

7. The method according to any of the preceding claims, wherein said modified nucleic acid target is detected by rolling circle amplification.

8. A method of determining an infectious disorder in a subject, said method comprising identifying a microorganism in a sample from said subject by a method as defined in any one of claims 1 to 7, wherein the presence of said microorganism in said sample is indicative of said infectious disorder, and wherein said microorganism belongs to the *Plasmodium* genus or the *Mycobacterium* genus.

9. Use of a double stranded nucleic acid target for a type I topoisomerase of a pathogenic microorganism and not for a human type I topoisomerase, for diagnosing an infectious disorder, wherein said microorganism belongs to the *Plasmodium* genus or the *Mycobacterium* genus.

10. The method of Claim 8 or the use according to claim 9, wherein said infectious disorder is malaria or tuberculosis.

11. A method for evaluating the effect of an agent on a microorganism in a sample, said method comprising
i. providing a sample
ii. providing a double-stranded nucleic acid target specific for type I topoisomerase of said microorganism, and not for human type I topoisomerase,
iii. providing an agent,
iv. combining the sample of step i. and the nucleic acid target of step ii. with or without the agent of step iii.
v. detecting nucleic acid target that has been modified by type I topoisomerase of said microorganism with or without the agent,
wherein an agent capable of reducing the amount of modified nucleic acid target has an inhibitory effect on said microorganism,
and wherein said microorganism belongs to the *Plasmodium* genus or the *Mycobacterium* genus.

## Patentansprüche

1. Verfahren zum Identifizieren eines Mikroorganismus in einer Probe, wobei das Verfahren Folgendes umfasst:
i. Bereitstellen der Probe,
ii. Bereitstellen eines Doppelstrang-Nukleinsäuretargets, das spezifisch ist für Typ-I-Topoisomerase des Mikroorganismus und nicht für humane Typ-I-Topoisomerase,
iii. Mischen der Probe aus Schritt i. mit dem Nukleinsäuretarget aus Schritt ii.,
iv. Erkennen des Nukleinsäuretargets, das durch die Typ-I-Topoisomerase des Mikroorganismus modifiziert worden ist,
wobei das Vorliegen von Nukleinsäuretarget, das von Typ-I-Topoisomerase des Mikroorganismus modifiziert worden ist, den Mikroorganismus anzeigt und wobei der Mikroorganismus zur Gattung *Plasmodium* oder zur Gattung *Mycobacterium* gehört.

2. Verfahren nach Anspruch 1, wobei der Mikroorganismus ein krankheitserregender Mikroorganismus ist.

3. Verfahren nach einem der vorhergehenden Ansprüche, wobei der Mikroorganismus *Plasmodium falciparum* oder *Mycobacterium tuberculosis* ist.

4. Verfahren nach einem der vorhergehenden Ansprüche, wobei die Probe Blutplasma, Blutserum, Speichel, Urin, Zellabstrich oder Kot ist.

5. Verfahren nach einem der vorhergehenden Ansprüche, wobei das Nukleinsäuretarget ein Doppelstrang-DNA-Substrat ist.

6. Verfahren nach einem der vorhergehenden Ansprüche, wobei das modifizierte Nukleinsäuretarget durch Southern Blot, Polymerase-Kettenreaktion, RT-PCR, qPCR, RFLD, Primer-Extension, DNA-Array-Technologie, Rolling-Circle-Amplifikation erkannt wird.

7. Verfahren nach einem der vorhergehenden Ansprüche, wobei das modifizierte Nukleinsäuretarget durch Rolling-Circle-Amplifikation erkannt wird.

8. Verfahren zum Bestimmen einer infektiösen Erkrankung in einem Subjekt, wobei das Verfahren das Identifizieren eines Mikroorganismus in einer Probe von dem Subjekt über ein Verfahren nach einem der Ansprüche 1 bis 7 umfasst, wobei das Vorliegen des Mikroorganismus in der Probe die infektiöse Erkrankung anzeigt und wobei der Mikroorganismus zur Gattung *Plasmodium* oder zur Gattung *Mycobacterium* gehört.

9. Gebrauch eines Doppelstrang-Nukleinsäuretargets für eine Typ-I-Topoisomerase eines krankheitserregenden Mikroorganismus und nicht für eine humane Typ-I-Topoisomerase, zum Diagnostizieren einer infektiösen Erkrankung, wobei der Mikroorganismus zur Gattung *Plasmodium* oder zur Gattung *Mycobacterium* gehört.

10. Verfahren nach Anspruch 8 oder Gebrauch nach Anspruch 9, wobei die infektiöse Erkrankung Malaria oder Tuberkulose ist.

11. Verfahren zum Auswerten der Auswirkung eines Wirkstoffs auf einen Mikroorganismus in einer Probe, wobei das Verfahren Folgendes umfasst:
i. Bereitstellen der Probe,
ii. Bereitstellen eines Doppelstrang-Nukleinsäuretargets, das spezifisch ist für Typ-I-Topoisomerase des Mikroorganismus und nicht für humane Typ-I-Topoisomerase,
iii. Bereitstellen eines Wirkstoffs,
iv. Kombinieren der Probe aus Schritt i. und des Nukleinsäuretargets aus Schritt ii. mit dem oder ohne den Wirkstoff aus Schritt iii.,
v. Erkennen des Nukleinsäuretargets, das durch die Typ-I-Topoisomerase des Mikroorganismus mit dem oder ohne den Wirkstoff modifiziert worden ist,
wobei ein Wirkstoff, der in der Lage ist, die Menge von modifiziertem Nukleinsäuretarget zu verringern, eine hemmende Wirkung auf den Mikroorganismus aufweist,
und wobei der Mikroorganismus zur Gattung *Plasmodium* oder zur Gattung *Mycobacterium* gehört.

## Revendications

1. Procédé d'identification d'un micro-organisme dans un échantillon, ledit procédé comprenant les étapes consistant à :
i. fournir l'échantillon,
ii. fournir un acide nucléique bicaténaire cible spécifique pour la topoisomérase de type I dudit micro-organisme, et non pour la topoisomérase humaine de type 1,
iii. mélanger l'échantillon de l'étape i. avec l'acide nucléique cible de l'étape ii.,
iv. détecter l'acide nucléique cible qui a été modifié par la topoisomérase de type I dudit micro-organisme,
dans lequel la présence d'acide nucléique cible modifié par la topoisomérase de type I dudit micro-organisme est révélatrice dudit micro-organisme,
et dans lequel ledit micro-organisme appartient au genre *Plasmodium* ou au genre *Mycobacterium.*

2. Procédé selon la revendication 1, dans lequel ledit micro-organisme est un micro-organisme pathogène.

3. Procédé selon l'une quelconque des revendications précédentes, dans lequel ledit micro-organisme est *Plasmodium falciparum* ou *Mycobacterium tuberculosis.*

4. Procédé selon l'une quelconque des revendications précédentes, dans lequel ledit échantillon est du plasma sanguin, du sérum sanguin, un crachat, de l'urine, un frottis cellulaire ou des matières fécales.

5. Procédé selon l'une quelconque des revendications précédentes, dans lequel ledit acide nucléique cible est un ADN substrat bicaténaire.

6. Procédé selon l'une quelconque des revendications précédentes, dans lequel ledit acide nucléique cible modifié est détecté par buvardage de Southern, amplification en chaîne par polymérase, RT-PCR, qPCR, RFLD, extension d'amorce, technologie des puces ADN, amplification par cercle roulant.

7. Procédé selon l'une quelconque des revendications précédentes, dans lequel ledit acide nucléique cible modifié est détecté par amplification par cercle roulant.

8. Procédé de détermination d'une maladie infectieuse chez un patient, ledit procédé comprenant l'identification d'un micro-organisme dans un échantillon dudit patient par un procédé selon l'une quelconque des revendications 1 à 7, dans lequel la présence dudit micro-organisme dans ledit échantillon est révélatrice de ladite maladie infectieuse, et dans lequel ledit micro-organisme appartient au genre *Plasmodium* ou au genre *Mycobacterium.*

9. Utilisation d'un acide nucléique bicaténaire cible spécifique pour une topoisomérase de type I d'un micro-organisme pathogène, et non pour une topoisomérase humaine de type I, pour le diagnostic d'une maladie infectieuse, dans laquelle ledit micro-organisme appartient au genre *Plasmodium* ou au genre *Mycobacterium.*

10. Procédé selon la revendication 8 ou utilisation selon la revendication 9, dans lequel ou dans laquelle ladite maladie infectieuse est le paludisme ou la tuberculose.

11. Procédé d'évaluation de l'effet d'un agent sur un micro-organisme dans un échantillon, ledit procédé comprenant les étapes consistant à :
i. fournir un échantillon,
ii. fournir un acide nucléique bicaténaire cible spécifique pour la topoisomérase de type I dudit micro-organisme, et non pour la topoisomérase humaine de type I,
iii. fournir un agent,
iv. mélanger l'échantillon de l'étape i. et l'acide nucléique cible de l'étape ii. avec ou sans l'agent de l'étape iii.,
v. détecter l'acide nucléique cible qui a été modifié par la topoisomérase de type I dudit micro-organisme avec ou sans l'agent,
dans lequel un agent capable de réduire la quantité d'acide nucléique cible modifié a un effet inhibiteur sur ledit micro-organisme,
et dans lequel ledit micro-organisme appartient au genre *Plasmodium* ou au genre *Mycobacterium.*
